# EUROPÄISCHE PATENTSCHRIFT

(11) **EP 1 410 448 B1**
(45) Veröffentlichungstag und Bekanntmachung des Hinweises auf die Patenterteilung: **25.03.2015**
(21) Anmeldenummer: 01967061.1
(22) Anmeldetag: 30.08.2001
(51) Int. Cl.: H01L 51/50, H01L 51/30, C07D 305/06, C07D 409/14, C07D 409/12, H01L 51/00

(54) **PHOTOSTRUKTURIERBARE NEUE ORGANISCHE HALBLEITERMATERIALIEN**
NOVEL PHOTOSTRUCTURABLE ORGANIC SEMICONDUCTOR MATERIALS
NOUVEAUX MATERIAUX SEMI-CONDUCTEURS ORGANIQUES PHOTOSTRUCTURABLES

(30) Priorität: 11.09.2000 DE 10044840
(43) Veröffentlichungstag der Anmeldung: 21.04.2004
(73) Patentinhaber: OSRAM Opto Semiconductors GmbH, 93055 Regensburg (DE)
(72) Erfinder: KANITZ, Andreas, 91315 Höchstadt (DE); ROGLER, Wolfgang, 91096 Möhrendorf (DE)
(74) Vertreter: Epping - Hermann - Fischer
(86) Internationale Anmeldenummer: PCT/DE2001/003346
(87) Internationale Veröffentlichungsnummer: WO 2002/021611

(56) Entgegenhaltungen:
- EP-A- 0 728 790
- EP-A- 0 848 294
- WO-A-01/12745
- DE-A- 3 827 221
- DE-A- 19 913 543
- FR-A- 1 501 297
- GB-A- 2 305 919
- US-A- 5 463 084
- US-A- 5 518 824
- PATENT ABSTRACTS OF JAPAN vol. 1999, no. 69, 30. Juli 1999 (1999-07-30) & JP 11 106380 A (UBE), 20. April 1999 (1999-04-20)
- CHEMICAL ABSTRACTS, vol. 130, no. 14, 1999 Columbus, Ohio, US; abstract no. 183514r, Seite 753; Spalte 2; XP002188882 & JP 01 117074 A (JSR CO.) 22. Januar 1999 (1999-01-22)
- CHEMICAL ABSTRACTS, vol. 135, no. 5, 2001 Columbus, Ohio, US; abstract no. 68558z, Seite 1402; Spalte 2; XP002188883 & JP 2001 174986 A (HITACHI CHEMICAL) 29. Juni 2001 (2001-06-29)
- CHEMICAL ABSTRACTS, vol. 135, no. 24, 2001 Columbus, Ohio, US; abstract no. 345501s, Seite 831; XP002188884 & JP 2001 303014 A (HITACHI CHEM.) 31. Oktober 2001 (2001-10-31)

## Beschreibung

Die Erfindung betrifft neue organische Halbleitermaterialien mit verbesserten Verarbeitungseigenschaften speziell im Hinblick auf die Herstellung von organischen Licht emittierenden Leuchtdioden (OLEDs).

OLEDs haben einen Schichtaufbau, der aus einem transparenten Träger (Glas, Kunststoff), einer darauf befindlichen Anode (z.B. aus ITO (Indium-Tin-Oxid)) zumindest einer organischen halbleitenden Schicht und schließlich aus einer Kathode besteht.

Die Effektivität des OLED hängt erheblich davon ab, ob die halbleitende Schicht aus einer (Einschichtsystem) oder mehreren Schichten (Mehrschichtsystem) aufgebaut ist. Die Mehrschichtsysteme sind effektiver und emittieren stärker. Zur Bildung der bevorzugten Mehrschichtsysteme eignen sich niedermolekulare Substanzen besser als polymere, weil bestehende polymere Schichten durch Aufbringung von angrenzenden polymeren Schichten mittels Spin-coating, Gießen oder Rakeln verändert und beschädigt werden. Selbst beim Einsatz sogenannter orthogonaler Lösungsmittel, in denen die bereits hergestellten Schichten unlöslich sind, wird normalerweise die Oberfläche angelöst und/oder angequollen. Dadurch werden die Moleküle der verschiedenen halbleitenden Schichten, also die Lochtransport- und/oder Elektronentransportmoleküle unter Umständen vermischt und/oder an der Grenzschicht ausgedünnt, was je nach Anwendung ein Nachteil (Reduktion der Lebensdauer und Effizienz) sein kann. Diese Problematik wird in der Literatur beschrieben, wie beispielsweise in der US-Patentschrift 4539507.

Andererseits sind die polymeren halbleitenden Materialien gegenüber den niedermolekularen halbleitenden Materialien bevorzugt, weil sie die höhere Glasübergangstemperatur und damit die geringere Kristallisationstendenz haben.

Zwar ist die Vernetzung und die Erhöhung der Glasübergangstemperatur des organischen Halbleiters auf dem Substrat beispielsweise aus der DE 4325885 A1 bekannt, jedoch birgt die Vernetzung einer Schicht in, auf einem elektronischen und/ oder optischen Prozess basierenden, Anordnungen auch Probleme in sich: Rissbildungen in den Filmen während der Vernetzung und/oder einzelne, bei der Vernetzung nicht umgesetzte polymerisierbare funktionelle Gruppen in der Schicht. Rissbildung tritt bei der Vernetzung insbesondere dann auf, wenn die Vernetzungsreaktion von einer großen Volumenschrumpfung des Materials begleitet wird.

Der Umfang der Volumenschrumpfung hängt unter anderem von der Art der eingesetzten polymerisierbaren Funktion und/oder von der Struktur der noch nicht vernetzten Verbindung ab. Von der Art und/oder der Reaktivität der bei der Vernetzung eingesetzten polymerisierbaren funktionellen Gruppe hängt insbesondere auch ab, wie hoch die Konzentration dieser Gruppe nach der Vernetzung im Material ist und/oder wie sich nicht umgesetzte Gruppen bei der Anwendung in der OLED-Anordnung auswirken.

In den literaturbekannten Anwendungen konnte durch die Vernetzung auf dem Substrat bisher keine Verbesserung der OLED-Eigenschaften erreicht werden. Mögliche Gründe hierfür wurden gerade aufgeführt, beispielsweise Rißbildung. Gerade bei der elektrischen und/oder optischen Anwendung können nicht umgesetzte polymersierbare Gruppen aber auch als Störstellen fungieren, die die Effektivität und/oder die Lebensdauer einer derartigen Anordnung stark herabsetzen. Daher wirken sich zu geringe Umsätze und/oder zu langsame Reaktionsgeschwindigkeit der eingesetzten polymerisierbaren funktionellen Gruppe gerade bei elektrischen und/oder optischen Anwendungen äußerst negativ aus.

So spielt bei einer auf einem elektrischen und/oder optischen Prozess basierenden Anordnung wie einer OLED, in der eine vernetzte Schicht enthalten ist, die Art der bei der Vernetzung eingesetzten polymerisierbaren Gruppe eine enorm wichtige Rolle.

Aufgabe der vorliegenden Erfindung ist es deshalb, ein organisches halbleitendes Material zu schaffen, das eine funktionelle Gruppe hat, die eine Erhöhung der Glasübergangstemperatur der unvernetzten Verbindung auf dem Substrat durch Vernetzung und eine gute Verarbeitbarkeit als Mehrschichtsystem bewirkt, wobei der Halbleiter die oben genannten Nachteile bei der Vernetzung auf dem Substrat wie Rissbildung durch Volumenschrumpfung, geringe Reaktionsgeschwindigkeit und unvollständige Umsetzung nur in geringem Umfang zeigt. Außerdem ist es Aufgabe der Erfindung, ein Verfahren zur Herstellung einer halbleitenden und/oder lumineszierenden Schicht anzugeben. Gegenstand der Erfindung ist ein niedermolekularer, oligomerer oder (pre)polymerer Halbleiter mit Oxetan-funktionalisiertem Substituenten, wobei der Halbleiter ladungstransportierende und/oder lumineszierende Eigenschaften hat und durch thermische Behandlung und/oder durch Bestrahlung vernetzbar ist.

Zudem ist Gegenstand der Erfindung ein Verfahren zur Herstellung eines niedermolekularen, oligomeren oder (pre)polymeren Halbleiters mit Oxetan-funktionalisiertem Substituenten durch Kondensation und/oder HX-Eliminierung nach folgendem allgemeinen Reaktionsschema: 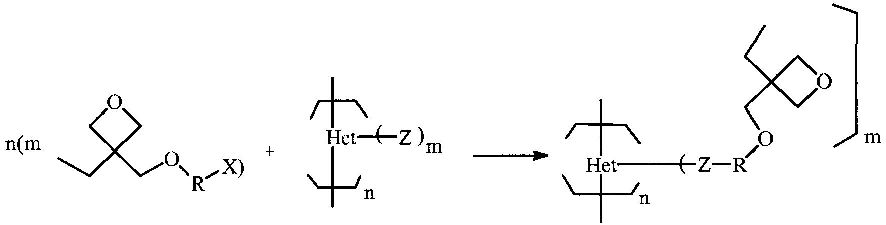 wobei für **Het, Z, R, X, n** und **m** gilt:
**Het** sind die heterocyclischen Grundkörper der ladungstransportierenden Halbleiter,
**Z** sind die Substituenten **R³** und/oder **R⁴** der heterocyclischen Grundkörper, die unabhängig voneinander oder beide eine Funktion tragen, die zur Anknüpfung an den Spacer **R** geeignet ist,
vorzugsweise sind dies Hydroxyphenyl- oder Aminophenylgruppierungen,
die Gruppe der einsetzbaren Spacer **R** umfasst verzweigte oder unverzweigte, unsubstituierte Alkylenketten, vorzugsweise C₁ bis C₈ -Ketten, und/oder etherhaltige Alkylenketten und/oder aromatenhaltige Alkylenketten.

**X** ein Halogen oder OH, je nach dem angewandten Reaktionstyp zur Verknüpfung von **Z** mit **R und**
die Gruppe **Z-Het** ist ausgewählt aus der Gruppe, die folgende Verbindungen umfasst:
2-Aminothiophenderivate **II** und **IIa**
5-akzeptor-funktionalisierte 2-N,N-Diarylamino-thiophene **IV** 2,3-Bishtienylchinoxaline **VII**
1,4-Bis-1,1'-dithienyl-2,2'-diaryl-bzw.-2,2,2',2'-tetraaryl-styrylbenzene **IX**
1,1-Dithienyl-2,2-diakzeptor-funktionalisierte Ethylene **X**
1-Thienyl-1-methyl-2,2-diakzeptor-funktionalisierte Ethylene **XI** und
4,6-bisthienyl-funktionalisierte 1,3,2-Dioxaborine **XII.**

Der Parameter **n** ist 1 für die vernetzungsfähigen monomeren Halbleiter und kann aus reaktionskinetischen Gründen für die oligomeren und polymeren Halbleiter maximal 100 erreichen. Der Parameter **m** kann 1 oder 2 sein.

Zudem sind Gegenstände der Erfindung Halbleiter mit der allgemeinen Struktur 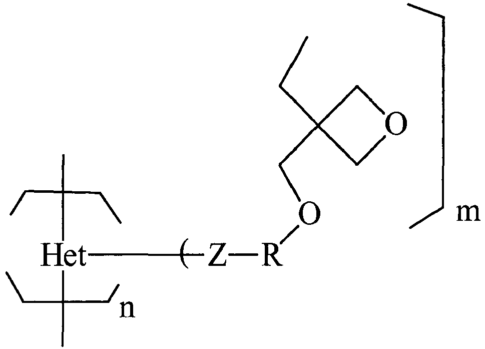 für diese die Symbole **R, Z** und **Het** die oben angeführte Bedeutung besitzen, wobei das Symbol **R¹** im Baustein **Het** zwingend die angegebene bifunktionelle Bedeutung hat; der Polymerisationsgrad **n,** der durch die Stufenkondensation bedingt einen Wert von n = 2 bis n = 100 erreichen kann und der Parameter **m,** der den Wert von 1 oder 2 annehmen kann.

Außerdem ist Gegenstand der Erfindung ein Verfahren zur Herstellung einer halbleitenden und/oder lumineszierenden Schicht durch Vernetzung eines niedermolekularen, oligomeren oder (pre)polymeren Halbleiters mit Oxetan-funktionalisiertem Substituenten.

Zudem ist Gegenstand der Erfindung eine organische Licht emittierende Diode (OLED), umfassend zumindest ein Substrat, eine Anodenschicht, eine Lochtransportschicht, eine Emitterschicht, die gleichzeitig auch eine Elektronentransportschicht sein kann, eine Kathode und eine Verkapselung, wobei die Lochtransportschicht und/oder die Elektronentransportschicht/Emitterschicht durch Vernetzung eines niedermolekularen, oligomeren oder (pre)polymeren Halbleiters mit Oxetan-funktionalisiertem Substituenten erhältlich ist.

Oxetane zeichnen sich durch hohe Reaktionsgeschwindigkeiten und/oder hohe Umsätze bei der Vernetzung in Substanz aus und/oder unterscheiden sich von anderen funktionellen Gruppen durch extrem niedrige Volumenschrumpfung während der Vernetzung. Die Inertheit der Oxetangruppe gegenüber stark basischen Reaktionsbedingungen ermöglicht ferner einen guten synthetischen Zugang zu neuen organischen Halbleitern mit ladungstranportierenden und/oder lumineszierenden Eigenschaften die peripher durch mindestens eine Oxetangruppe substituiert sind. So können beispielsweise oxetanfunktionalisierte Edukte in effektiven Synthesen problemlos eingesetzt werden.

Durch die Vernetzung auf dem Substrat erhöht der Halbleiter mit Oxetan-funktionalisiertem Substituenten seine Glasübergangstemperatur und/oder wird unlöslich, so dass ein Mehrschichtaufbau für eine OLED, die bei Betriebstemperaturen oberhalb 100°C arbeitet, herstellbar ist.

Die Vernetzung der Oxetane erfolgt nach einem kationischen Mechanismus und wird bevorzugt durch thermische Initiatoren, z.B. thermisch latente Lewis-Säuren, und/oder durch Photoinitiatoren durch Bestrahlung mit aktinischer Strahlung der Wellenlänge 200nm bis 600nm, z.B. Photosäuren, initiiert.

Dabei kann das Oxetan an den niedermolekularen Halbleiter und/oder an das Polymerrückrat und/oder an die Seitenkette eines Halbleiters unmittelbar und/oder über einen Spacer kovalent angebunden sein. Der Halbleiter mit ladungstransportierenden Eigenschaften kann eine niedermolekulare, oligomere und/oder polymere Verbindung sein. Der Grundkörper des bevorzugten Halbleiters mit Oxetan-funktionalisiertem Substituenten basiert auf heterocyclisch substituierten tertiären aromatischen Aminen, die durch bekannte Kondensationsreaktionen nach einem Baukastenprinzip darstellbar sind und aufgrund ihres Substitutionsmusters Elektronen oder Löcher transportieren können und zur Elektrolumineszenz im Bereich des sichtbaren elektromagnetischen Spektrums befähigt sind. Zur Steigerung der Lumineszenz in den OLEDs sind weiterhin alle, aus Laseranwendungen bekannten Fluoreszenzfarbstoffe prinzipiell einsetzbar.

Bevorzugt eingesetzt werden als Halbleiter mit Oxetan-funktionalisiertem Substituenten die Ladungstransportmaterialien (Charge Transport Materials) **CTM,** welche durch die folgende Struktur charakterisiert sind: 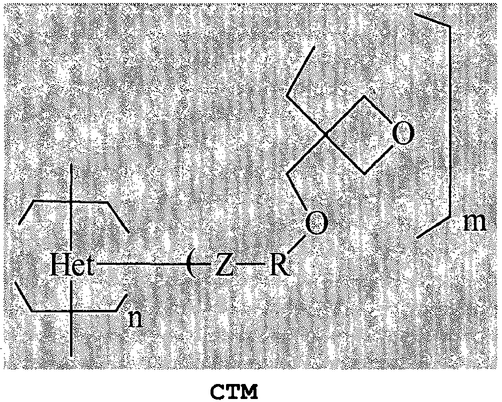 für die folgendes gilt:
**Het** sind die heterocyclischen Grundkörper der ladungstransportierenden Verbindungen,
**Z** sind die Substituenten **R³** und/oder **R⁴** der heterocyclischen Grundkörper, die unabhängig voneinander oder beide eine Funktion tragen, die zur Anknüpfung an den Spacer **R** geeignet ist, vorzugsweise sind dies Hydroxyphenyl- oder Aminophenylgruppierungen,
   die Gruppe der einsetzbaren Spacer **R** umfasst verzweigte oder unverzweigte unsubstituierte Alkylenketten, vorzugsweise C₁ bis C₈-Ketten, und/oder etherhaltige Alkylenketten und/oder aromatenhaltige Alkylenketten,
**X** ist Halogen oder OH, je nach angewandten Reaktionstyp zur Verknüpfung von **Z** mit **R.**

Bevorzugt werden die ladungstransportierenden heterocyclischen Materialien **Z-Het (II; IIa; IV; VII; IX; X; XI; und XII)** in einfachen Syntheserouten sämtlich aus Thiocarbonsäureamiden des Typs **I** gemäß dem folgenden Reaktionsschema zugänglich, eingesetzt, die einen Polymerisationsgrad **n,** der durch die Stufenkondensation bedingt einen Wert von n = 2 bis n = 100 erreichen können und deren Parameter **m,** den Wert von 1 oder 2 annehmen kann.

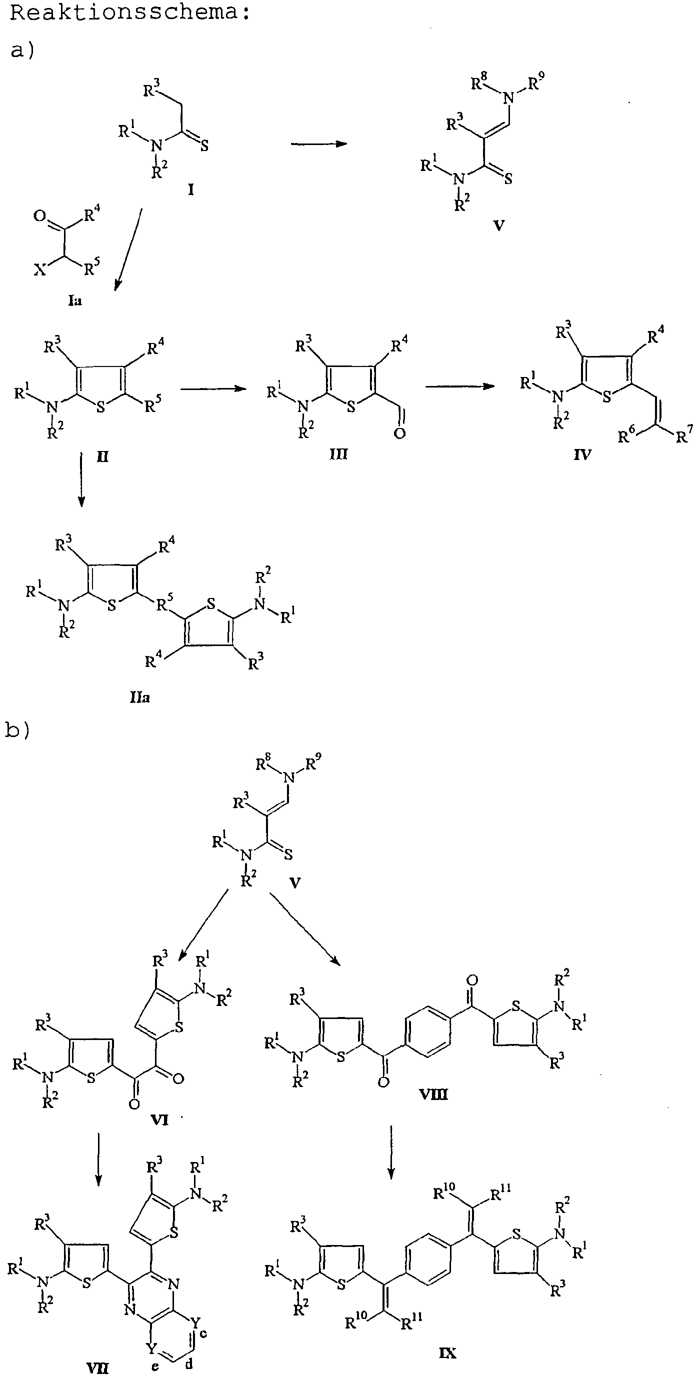

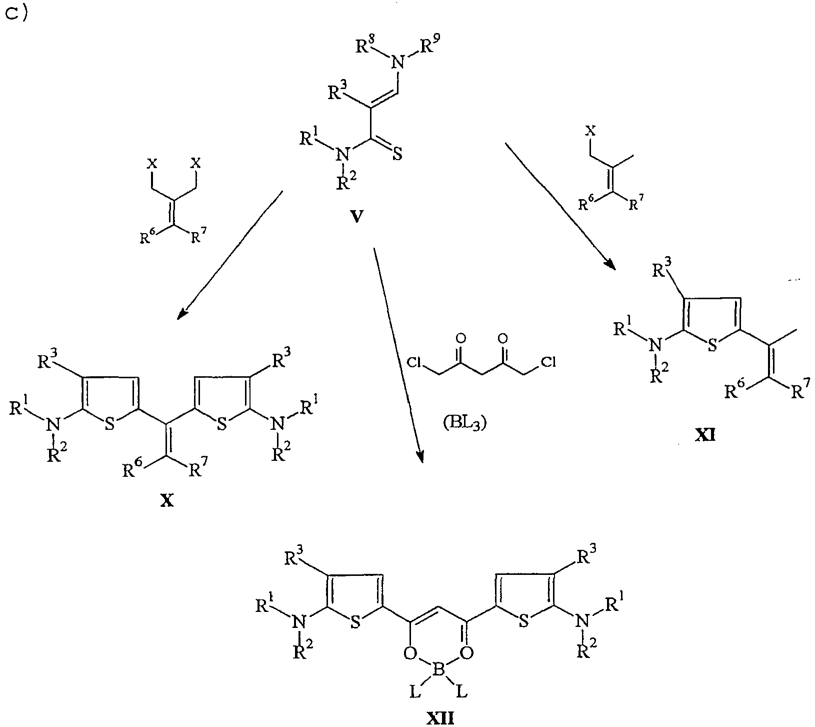

### Synthesebeschreibung:

a) Synthese der Lochtransportmaterialien **II** und **IIa:**
   Aus den Thiocarbonsäureamiden des Typs **I** und den 2-Halogenacylverbindungen **Ia** synthetisiert man die N,N-disubstituierten 2-Aminothiophenderivate **II** durch eine Hantzsch-analoge Alkylierungs- und Kondensationsreaktion. Aus den 5-unsubstituierten 2-Aminothiophenderivaten II werden die 5,5'-Bisthienylderivate IIa durch Oxydation aber auch durch lithium-organische Kupplungsreaktionen erhalten.
b) Die orange bis rot emittierenden Elektronentransportmaterialien **IV** werden erhalten, wenn man bei der Hantzschanalogen Reaktion 2-Halogenacylverbindungen **Ia** mit dem Substituenten R⁵=H einsetzt. Die sich dabei bildenden Materialien des Typs **II** werden durch eine Vilsmeier Reaktion in die N,N-disubstituierten 2-Amino-5-formyl-thiophene **III** überführt, die danach in einem weiteren Kondensationsschritt mittels methylen-aktiver Verbindungen zu hocharylierten Emittern des Typs **IV** reagieren.
c) Die Thiocarbonsäureamide **I** sind gleichzeitig Edukte zur Bildung von vinylogen Thiocarbonsäureamiden **V,** aus welchen weitere Emittermaterialien zugänglich sind.
d) Diese Verbindungen **V** können mit 1,4-Dibrombutan-2,3-dion zu 1,2-Diketonen des Typs **VI** umgewandelt werden. Aus diesen wiederum erhält man bei der Reaktion mit o-Aminoarylverbindungen einen neuen gelb bis orangen Emittertyp, die 2,3-Bisthienylchinoxaline **VII.**
e) Durch Umsetzung der Verbindungen des Typs **V** mit 1,4-Dibromacetylbenzen bilden sich die Diketone des Typs **VIII.** In einer Horner-Wittig Reaktion mit Derivaten, die durch eine Michaelis-Arbusov Reaktion zugänglich sind, entstehen aus den Ketonen **VIII** blaue bis gelbe Emittermaterialien des Typs **IX.**
f) In einer Reaktion der vinylogen Thiocarbonsäureamide **V** mit 1,1-Dihalomethyl-2,2-diakzeptor-substituierten Ethylenen erhält man rot emittierende Emittermaterialien des Typs **X.**
g) Ebenfalls orange bis rot emittierende Emittermaterialien **XI** werden beim Umsatz von Verbindungen des Typs **V** mit 1-Halomethyl-1-methyl-2,2-diakzeptor-substituierten Ethylenen erhalten.
h) Aus den Verbindungen des Typs V können schließlich mit 1,5-Dichloracetylaceton und anschließender Komplexierung mittels Borsäurederivaten die hocheffizient rot emittierenden Emittermaterialien des Typs XII, die 4,6-bisthienyl-substituierten 1,3,2-Dioxaborine erschlossen werden.

Die variablen Substituenten der im Reaktionsschema auftretenden Zwischen- und Endprodukte haben die folgende Bedeutung: **R¹, R²,** und **R⁵** sind - unabhängig voneinander - jeweils ein monofunktionelles (Het)arylsystem, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylamiogruppen (Alkyl = C₁ bis C₆) substituiert sein können;
folgende (Het)arylsysteme sind dabei bevorzugt:
Phenyl-, Biphenyl-, Alkylphenyl-, Alkoxyphenyl-, Phenoxyphenyl-, Naphthyl-, Anthryl-, Phenanthryl-, 4-Tritylphenyl-, Thienyl,- Thiazolyl-, Benzothiazolyl-, Pyridyl-, Chinolyl-, Isochinolyl-, Pyridazinyl-, Pyrimidinyl-, Chinazolyl-, Pyrazinyl-, Chinoxalyl-, Phenazinyl- und Pyrenylsysteme.

**R¹** kann ferner ein entsprechendes bifunktionelles (Het)arylensystem sein, d.h. ein konjugiertes carbocyclisches oder heterocyclisches Ringsystem, das auch aus linear oder angular anellierten oder verknüpften gleichen oder unterschiedlichen Ringtypen bestehen kann, wobei die peripheren Wasserstoffatome gegebenenfalls durch Alkyl-, Alkoxy-, Phenoxy-, Dialkylamino- oder Diphenylaminogruppen (Alkyl = C₁ bis C₆) substituiert sein können;
folgende (Het)arylensysteme sind dabei bevorzugt:
Phenylen-, Biphenylen-, Alkylphenylen-, Alkoxyphenylen-, Phenoxyphenylen-, Naphthylen-, Anthrylen-, Phenanthrylen-, Thienylen,- Thiazolylen-, Pyridazinylen-, Phthalazinylen- und Pyrazinylensysteme.

R¹ und R² kann auch gemeinsam eine 10-Phenothiazinyl-, 10-Phenoxazinyl oder 9-Carbazolylgruppierung bilden.

**R⁵** kann ferner H sein.

Ausschließlich in Verbindung **IIa** hat **R⁵** die Bedeutung einer chemischen Bindung.

**R³** und **R⁴** entsprechen der oben erklärten Variablen **Z,** für den Fall, dass **R³** oder **R⁴** nicht der funktionalisierbaren Variablen **Z** entspricht hat dieser Substituent die Bedeutung von **R²** oder Wasserstoff.

**R⁶** und **R⁷,** die gleich oder verschieden sein können, aber einfache oder komplexer gebaute Gruppierungen mit Elektronenakzeptorcharakter darstellen, die man gemeinsam mit dem beide Substituenten verbindenden C-Atom sämtlich als von der Malonsäure sowie deren cyclischer Ureide und Thioureide abgeleitete Gruppierungen auffassen kann; demnach **R⁶** und **R⁷** Nitril-Gruppierungen, Carbonsäureestergruppierungen, und Carbonsäureamid-gruppierungen sein können, wobei die Ester- Amid- bzw. Ureidreste verzweigte oder unverzweigte Alkylreste mit bis zu 10 Kohlenstoffatomen umfassen können, 4-Triphenylmethylphenylreste (4-Tritylphenylreste) sowie R² sein können.

**R⁸** und **R⁹** sind C₄- bis C₁₀-Alkylgruppierungen oder bilden gemeinsam eine C₄- bis C₆-Alkylengruppierung oder eine Gruppierung -CH₂-CH₂-O-CH₂-CH₂-.

**R¹⁰** und **R¹¹** haben unabhängig voneinander die Bedeutung von **R²,** ferner kann einer der beiden Substituenten auch H sein. **R¹⁰** und **R¹¹** können auch mit dem beide Substituenten verbindenden Kohlenstoffatom einen 9-Fluorenylidenrest bilden.

**Y** kann Kohlenstoff oder Stickstoff sein. Für den Fall, dass **Y**=C ist können unabhängig voneinander an den Bindungen c, d und e weitere aromatische Ringstrukturen anelliert sein; Für den Fall, dass **Y**=N ist können an der Bindung d weitere aromatische Ringstrukturen anelliert sein und schließlich die

Komplexliganden L die, unabhängig voneinander gleich oder verschieden sein können, stehen für Halogen, dabei vorzugsweise Fluor, Alkyl- oder Aryloxy, wobei die Alkylgruppierungen verzweigt oder unverzweigt 1 bis 20 C-Atome und verschiedene funktionelle Gruppen mit N, O und/oder S enthalten können oder beide gemeinsam, durch einen zumindest zweizähnigen Liganden mit dem Boratom einen Cyclus bilden, wobei der zumindest zweizähnige Ligand Hydroxy- und/oder Carbonsäuregruppen haben kann und vorzugsweise ein Diol, eine Hydroxycarbonsäure oder eine Dicarbonsäure, wie -OC₂H₄O-,-OC₃H₆O-, Glykolat, Laktat, Tartrat, Sylicylat, Mandelat, Benzilat, 1,2- oder 2,3-Hydroxynaphthoat, Oxalat, Malonat, Alkylmalonat oder Dialkylmalonat ist.

Die Anbindung des Spacers **R** kann demnach bevorzugt über Phenolether, Benzylether, Phenolthioether und/oder tertiäre Amine (aliphatisch und/oder aromatisch) erfolgen.

Ein besonderer Vorteil der oben beschriebenen Synthese der Materialien für die halbleitende und/oder lumineszierende Schicht sowie der Herstellungsmethode für die Schicht selbst ist, dass in keinem Verfahrens(teil)schritt eine Metallkatalyse erforderlich ist. Der Einsatz von metallkatalysierten Verfahrens(teil)schritten führt häufig zu einem Verlust oder zur Zerstörung der lumineszierenden Eigenschaften.

Gemäß einer Ausführungsform erfolgen die Herstellung der halbleitenden und/oder lumineszierenden Schicht und die Synthese der Materialien durch metallkatalysefreie Synthesemethoden.

Die Länge und/oder Art der eingesetzten Spacer beeinflussen die Materialeigenschaften wie beispielsweise Glasübergangstemperatur, Filmbildungseigenschaften, Kristallisationsverhalten, Löslichkeit, Reaktivität und/oder der maximal erreichbare Umsatz der unvernetzten Verbindungen. Im Netzwerk können unter anderem die Ladungstransporteigenschaften und/ oder die Materialeigenschaften über die Spacereigenschaften variiert und/oder optimiert werden.

Die Materialeigenschaften der unvernetzten Halbleiter mit Oxetan-funktionalisierten Substituenten sind von besonderer Wichtigkeit für die Verarbeitbarkeit. Insbesondere die Löslichkeits-, Filmbildungs- und/oder Kristallisationseigenschaften und/oder die Glasübergangstemperatur. Deshalb können niedermolekulare und/oder oligomere und/oder polymere Halbleiter mit Oxetan-funktionalisiertem Substituenten mit ladungstransportierenden Eigenschaften verwendet werden. Ausdrücklich seien auch dimere Verbindungen erwähnt, in denen zwei ladungstransportierende Verbindungsteile über Brücken verbunden sind. Mit denselben Gruppen kann man auch zu höheren Homologen gelangen.

Neben niedermolekularen Halbleitern mit ladungstransportierenden Eigenschaften mit zwei oder mehreren Oxetan-funktionalisierten Substituenten können auch niedermolekulare Halbleiter mit ladungstransportierenden Eigenschaften mit einem Oxetan-funktionalisiertem Substituenten zu den Halbleitern mit zwei oder mehreren Oxetan-funktionalisierten Substituenten zugesetzt werden. Diese Halbleiter umfassen einen und/oder mehrere ladungstransportierende Verbindungsteile, an die nur eine Oxetanfunktion kovalent angebunden ist. Diese Halbleiter mit einem Oxetan-funktionalisiertem Substituenten wirken als Reaktivverdünner und können durch ihre höhere Mobilität zu hohen Umsätzen an Oxetan führen und somit zur Umsatzmaximierung beitragen, ohne die Konzentration der Halbleiter mit ladungstransportierenden Eigenschaften im Netzwerk herabzusetzen.

Die Halbleiter mit ladungstransportierenden Eigenschaften mit einem Oxetan-funktionalisiertem Substituenten können auch durch Verwendung von polymeren und/oder nierdermolekularen mehrfach oxetanfunktionalisierten Verbindungen, die keine ladungstransportierenden Eigenschaften besitzen und/oder als Vernetzer wirken, in ein Netzwerk eingebunden werden. Es tragen insbesondere polymere Vernetzer zur Verbesserung der Filmbildungseigenschaften der noch unvernetzten Schichten bei. Die polymeren Vernetzer können aus einem Polymerrückrat bestehen, das beispielsweise aus Polystyrol, Poly(α-methylstyrol), Polyacrylat, Polymethacrylat, Polyester, Polyamid und/oder Polysulfonat besteht, und teilweise und/oder vollständig oxetansubstituiert ist.

Die Erfindung umfasst auch Polymere mit Oxetan-funktionalisiertem Substituenten mit ladungstransportierenden Eigenschaften. Diese Polymere eignen sich wegen ihrer guten Filmbildungseigenschaften ebenfalls zur Schichtenpräparation für OLED-Anordnungen. Auch Kombinationen von niedermolekularen Halbleitern mit Oxetan-funktionalisiertem Substituenten mit ladungstransportierenden Eigenschaften und polymeren Halbleitern mit Oxetan-funktionalisiertem Substituenten mit ladungstransportierenden Eigenschaften in einer oder mehreren Schichten sind möglich.

Zur Herstellung der halbleitenden und/oder lumineszierenden Schicht wird ein geeignetes Substrat (z.B. Glas, Folie, unlösliches organisches transparentes Material) durch ein Verfahren aus Lösung und/oder aus Substanz mit dem Halbleiter mit Oxetan-funktionalisiertem Substituenten beschichtet, vorzugsweise durch Spin-coating oder Rakeln aus verdünnter Lösung. Die Lösung umfasst neben dem Halbleiter mit Oxetan-funktionalisiertem Substituenten optional weitere Komponenten, wie Bindemittel, Reaktivverdünner, Vernetzer und/oder thermischen Initiator und/oder Photoinitiator. Nach eventueller Trocknung wird der entstandene Film thermisch und/oder mit aktinischer Strahlung polymerisiert und/oder vernetzt. Anschließend kann sogleich die nächste Schicht aufgebracht werden. Durch die Vernetzung bildet sich ein unlösliches kovalentes Netzwerk aus, bei dem auch bei Auftragung der nächsten Schicht aus Lösung (z.B. Spin-coating, Rakeln) und/oder aus Substanz keine Vermischung der bereits aufgetragenen Schicht mit der frisch aufgetragenen Schicht auftritt.

Mittels strahlungsinduzierter Vernetzung können die Schichten bzw. die Schicht durch bildgemäßes Belichten natürlich auch strukturiert werden. Dies dient erfindungsgemäß insbesondere für die Auftragung der verschiedenden Emittermaterialien für vollfarbige und/oder mehrfarbige Displayanwendungen zur Farbpixelierung der Displays. Eine Kombination von vernetzten Schichten mit unvernetzten Schichten, die durch Aufdampfen, Spin-coating oder Rakeln aufgetragen werden, ist natürlich auch möglich. Hier werden in der Regel bis auf die jeweils letzte alle anderen Schichten zuvor vernetzt worden sein.

Als Lösungsmittel eignen sich insbesondere cyclische Ether, wie z.B. substituierte Epoxide, substituierte und/oder unsubstituierte Oxetane und/oder Tetrahydrofuran.

Diese cyclischen Ether können für den Fall, dass das Lösungsmittel nicht vollständig durch Evakuieren entfernt werden kann, an der Vernetzungsreaktion als Reaktivverdünner mitwirken und somit zur Erhöhung des Umsatzes und/oder des Vernetzungsgrades der hergestellten Schichten beitragen, d.h. die Lösungsmittelreste werden als Co-Monomer im Laufe der Polymerisation bzw. Vernetzungsreaktion in das entstehende Polymer bzw. das Polymernetzwerk eingebaut.

Manche der Halbleiter mit Oxetan-funktionalisiertem Substituenten sind flüssig und/oder ölig und müssen daher nicht unbedingt in Lösungsmitteln gelöst werden, sondern können auch pur und/oder in Mischungen aus mehreren Monomeren ohne Lösungsmittel aufgetragen werden. Diese Mischungen können außerdem kationischen Initiator, Bindemittel, Reaktivverdünner und/oder zusätzliche Vernetzer umfassen und z.B. durch Spin-coating und/oder Rakeln zu Schichten verarbeitet werden. Die Verwendung der beschriebenen Schichten eignet sich auch zur Herstellung dickerer Schichten von über 10µm. Eine Verarbeitung kann beispielsweise auch durch Spritzgießen, durch Nutzung von Drucktechniken und/oder sogenannten "real to real"-Techniken erfolgen. Die erhaltenen Schichten sind thermisch stabil, unlöslich und mechanisch belastbar. Je nach gewünschter Ausführung können analog weitere Schichten oder sofort die zweite Elektrode aufgebracht werden. Derartige Schichtanordnungen zeichnen sich durch eine besonders gute thermische Belastbarkeit, hohen amorphen Charakter und Unlöslichkeit aus.

Die erfindungsgemäßen Schichten eignen sich insbesondere durch die Möglichkeit der Strukturierung der organischen Schichtmaterialien (z.B. thermisch und/oder Photostrukturierung) zum Aufbau von OLEDs ohne die Strukturierung mit Photolackschichten und/oder andere Hilfsbeschichtungen anwenden zu müssen.

Die Schichtdicken können je nach Anwendung von einigen Nanometern, bis hin zu Millimetern variieren. Ein bevorzugter Bereich ist von 25 bis 250 nm Schichtdicke. Beim Aufbau von OLEDs resultierten mit Schichtdicken unter 90 nm die effizientesten Anordnungen. Die Schichtdicke kann aber auch jeweils kleiner als die angegebene Untergrenze und/oder größer als die angegebene Obergrenze sein.

### Ausführungsbeispiele:

Zur analytischen Kontrolle wurden die synthetisierten Verbindungen durch NMR-Spektroskopie sowie durch ESI-Massenspektroskopie untersucht. Die UV/Vis- und fluoreszenzspektroskopischen Untersuchungen wurden in THF durchgeführt.

### a) Allgemeine Vorschrift zur Synthese der Thioamide I

In einem 2 1-Dreihalskolben mit Rückflusskühler, Magnetrührer, Tropftrichter und Inertgasdurchfluss wird jeweils 1 mol eines sekundären aromatischen Amins entsprechend der Substituentendefinition in 600 ml Dioxan gelöst. Das benötigte gemäß des Substituentenschlüssels substituierte Essigsäurechlorid, hier beispielhaft mit 4-O-Acetylphenyl-essigsäurechlorid, wird zuvor aus 4-O-Acetylphenylessigsäure und Thionylchlorid dargestellt, das überschüssige Thionylchlorid im Vakuum entfernt und danach in äquivalenter Menge der Aminlösung zugetropft. Anschließend wird das Reaktionsgemisch unter Rückfluss erhitzt, bis die gesamte Menge des bei der Reaktion entstehenden Chlorwasserstoffes vom Inertgasstrom entfernt worden ist. Durch dünnschichtchromatographische Kontrolle kann das Ende der Reaktion zusätzlich detektiert werden. Die Reaktionslösung wird dann abgekühlt und mindestens in das doppelte Volumen Eiswasser eingerührt. Dabei scheidet sich in den meisten Fällen ein Öl ab, welches mitunter erst nach einigen Stunden beim Rühren erstarrt. Die wässrige Phase wird abgetrennt und das Rohprodukt aus Acetanhydrid umkristallisiert.

Das auf diese Art erzeugte Carbonsäureamid wird wie folgt in ein Thioamid umgewandelt
0,5 mol des jeweiligen Carbonsäureamids und die äquivalente Menge Lawesson-Reagenz (hergestellt aus Anisol und Phosphorpentasulfid) werden in einer Rückflussapparatur mit Inertgasdurchfluss in 500 ml Dioxan suspendiert, und nachfolgend 6 h bei 100°C gerührt. Dabei bildet sich eine klare Lösung. Nach dünnschichtchromatographischer Kontrolle des Reaktionsendes wird die Reaktionsmischung in die doppelte Menge Wasser eingerührt, die sich oft bildende ölige Phase lässt man dann kristallisieren. Danach wird das Produkt von der wässrigen Phase abgetrennt und aus Acetanhydrid umkristallisiert. Die Ausbeute beträgt bezogen auf das Amin jeweils mindestens 60%.

### Beispiel 1

Auf diese Art wird das Thiocarbonsäureamid I/1 (R¹ = R² = Phenyl, R³ = 4-O-acetylphenyl) M⁺ = 361 hergestellt.

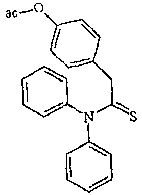

### Beispiel 2

Auf diese Art wird das Thiocarbonsäureamid I/2 (R¹ = 1,4-Phenylen, R² = Phenyl, R³ = 4-O-acetylphenyl) M⁺ = 644 hergestellt.

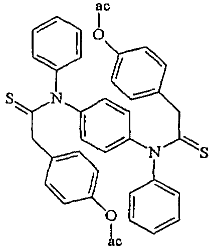

### b) Allgemeine Vorschrift zur Synthese der Aminothiophenderivate II

0,1 mol des jeweiligen Thiocarbonsäureamids I werden in einem Kolben, der mit Rührer und Rückflusskühler versehen ist, zusammen mit der äquivalenten Menge einer α-Halogenacylverbindung Ia in 200 ml Acetanhydrid gelöst, dann wird 1 h auf 120°C erwärmt. Nach dem Abkühlen wird das gebildete Aminothiophen-Derivat ausgefällt, indem es auf Eis gegossen wird und durch absaugen isoliert. Das Rohprodukt wird durch Lösen in THF und Ausfällen mit Ethanol gereinigt. Die Ausbeute beträgt jeweils zwischen 60 und 80%.

### Beispiel 3

Auf diese Art wird das Thiophen-Derivat II/1 (R¹ = R² = Phenyl, R³= = 4-O-acetylphenyl, R⁴ = Phenyl, R⁵ = H) M⁺ = 461) aus dem Thiocarbonsäureamid I/1 (R¹ = R² = Phenyl R³ = 4-O-acetylphenyl) und Phenacylchlorid hergestellt.

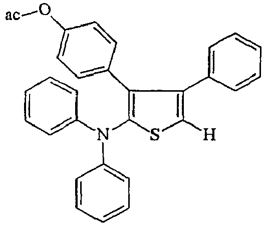

### Beispiel 4

Auf diese Art wird das Thiophen-Derivat II/2 (R¹ = 1,4-Phenylen, R² = Phenyl, R³ = 4-O-acetylphenyl, R⁴ = R⁵ = Phenyl) M⁺ - 996 aus dem Thiocarbonsäureamid I/2 (R¹ = 1,4 Phenylen, R² = Phenyl R³ = 4-O-acetylphenyl) und Desylchlorid (α-Chlor-α-phenylacetophenon) hergestellt.

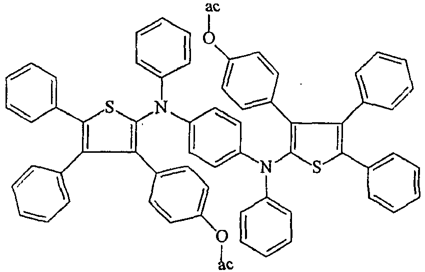

### c) Allgemeine Vorschrift zur Synthese von dimeren bzw. polymeren Aminothiophen-Derivaten IIa (durch oxidative Kupplung)

0,01 mol eines in 5-Stellung unsubstituierten Thiophen-Derivates II werden in einem Kolben, der mit Rückflusskühler, Rührer, Feststoffdosiereinrichtung und Inertgasdurchfluß versehen ist, in 100 ml getrocknetem THF gelöst. Es wird auf -60°C abgekühlt, und dann werden 0,015 mol Butyllithium zugesetzt. Anschließend wird die Kühlung entfernt und man lässt das Reaktionsgemisch bis auf -10°C auftauen. Nachfolgend werden mittels der Feststoffdosiereinrichtung 0,011 mol Kupfer-II-chlorid zugefügt, und dann wird weiter bis auf 40°C erwärmt. Die Reaktion wird nach 30 min abgebrochen, indem das Produkt mit Wasser (bei Polymeren mit Methanol mit einem Zusatz von 10% Wasser) ausgefällt und danach abgesaugt wird. Durch mehrfaches Lösen in THF und Ausfällen mit Methanol wird das Produkt gereinigt. Bei nichtpolymeren Verbindungen ist die Reinigung auch durch Umkristallisation aus Acetanhydrid möglich.

### Beispiel 5

Auf diese Art wird das dimere Thiophen-Derivat IIa/1 (R¹ = R² = R⁴ = Phenyl, R3 = 4-O-acetylphenyl) M⁺ = 920 aus Verbindung II/1 hergestellt.

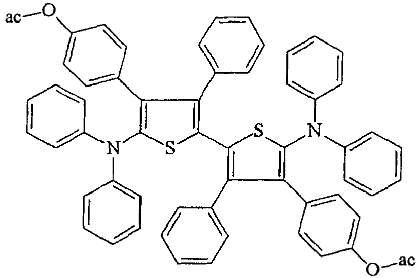

### d) Allgemeine Vorschrift zur Synthese der 2-Amino-5-formylthiophene III

In einem 250ml Becherglas mit Rührer werden 0,02 mol) einer 5-unsubstituierten Verbindung des Typs II in 50ml Dimethylformamid gelöst. Unter Kühlung auf etwa 5°C werden anschließend langsam 0,025 mol Phosphoroxychlorid POCl₃ hinzugegeben, danach lässt man noch 30 min rühren und erwärmt dabei auf etwa 30°C. Nach beendeter Reaktion wird vorsichtig in Eiswasser gegossen, wobei gelbe Kristalle ausfallen.

Das Rohprodukt wird abgesaugt und aus Ethanol umkristallisiert. Die Ausbeute beträgt mindestens 50%.

### Beispiel 6

Auf diese Art wird das 2-Amino-5-formyl-thiophene III/1 (R¹ = R² = R⁴ = Phenyl, R3 = 4-O-acetylphenyl) M⁺ = 489 aus Verbindung II/1 hergestellt.

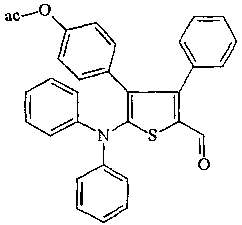

### e) Allgemeine Vorschrift zur Synthese der 5-akzeptorsubstituierten 2-Aminothiophene IV

In einem 250 ml Becherglas werden 0,01 mol eines 2-Amino-5-formylthiophens III und 0,015 mol einer entsprechenden methylenaktiven Akzeptorverbindung in 30 ml Acetanhydrid suspendiert und anschließend für einige Minuten unter Zusatz einiger Tropfen Triehylamin zum Sieden erhitzt. Nach beendeter Reaktion wird auf 100g Eis gegossen, wobei das Rohprodukt ausfällt. Es wird von der wäßrigen Phase abgetrennt und aus Acetanhydrid umkristallisiert. Die Ausbeute beträgt mindestens 30%.

### Beispiel 7

Auf diese Art wird das 5-akzeptor-substituierte 2-Aminothiophen IV/1 (R¹ = R² = R⁴ = Phenyl, R3 = 4-O-Acetylphenyl, R5 = 2,2-Dicyanovinyliden) M⁺ = 537, λₘₐₓ = 485nm, λ_{Φ} = 595 nm aus Verbindung III/1 und Malonsäuredinitril hergestellt.

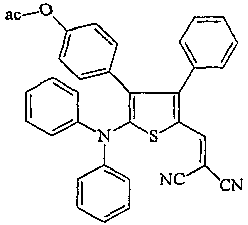

### Beispiel 8

Auf diese Art wird das 5-akzeptor-substituierte 2-Aminothiophen IV/2 (R¹ = 1,4-Phenylen, R² = R⁴ = Phenyl, R3 = 4-O-Acetylphenyl, R5 = N,N'-Diphenyl-5,5-vinyliden-thiobarbitursäure M⁺ = 1456, λₘₐₓ = 520nm, λ_{Φ} = 618 nm aus der entsprechenden 5,5'Bisformylverbindung des Typs III und N,N'-Diphenylthiobarbitursäure hergestellt.

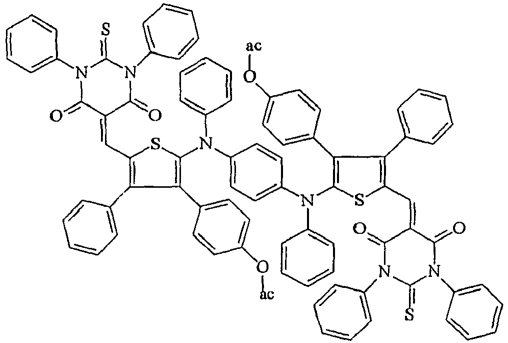

### f) Allgemeine Vorschrift zur Synthese der vinylogen Thiocarbonsäureamide V

In einem 0,5 l-Kolben mit Rückflusskühler, Heizeinrichtung und Rührer werden 0,15 mol 2-O-Acetylphenyl-thioessigsäurephenyl-1-naphthylamid zusammen mit der 4-fach äquivalenten Menge Morpholin und 4-fach äquivalenten Menge Orthoameisensäuretriethylester für 7h auf 125°C erwärmt. Nach beendeter Reaktion lässt man abkühlen und verdünnt mit Methanol wobei gelbe Kristalle ausfallen, die dann abgesaugt werden. Das Rohprodukt wird in Ethanol umkristallisiert. Die Ausbeute beträgt mindestens 60%.

### Beispiel 9

Auf diese Art wird das vinyloge Thiocarbonsäureamide V/1
(R¹ = Phenyl, R² = Naphthyl, R3 = 4-O-Acetylphenyl, R⁸,R⁹ bilden mit dem Stickstoff Morpholin M⁺ = 508 aus dem entsprechenden Thiocarbonsäureamid des Typs I, Orthotriethylformiat und Morpholin hergestellt.

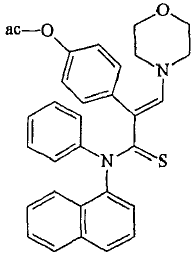

### Beispiel 10

Auf diese Art wird das vinyloge Thiocarbonsäureamide V/2
(R¹ = 1,4-Phenylen, R² = Phenyl, R3 = 4-O-Acetylphenyl, R⁸, R⁹ bilden mit dem Stickstoff Morpholin M⁺ = 838 aus dem entsprechenden Thiocarbonsäureamid des Typs I, Orthotriethylformiat und Morpholin hergestellt.

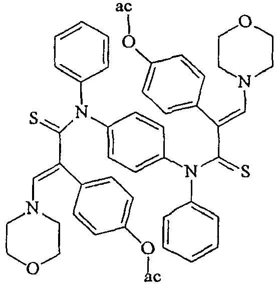

### g) Allgemeine Vorschrift zur Synthese der Bisthienyl-1,2-dicarbonyl-verbindungen VI

In einem Kolben mit Rückflusskühler, Heizeinrichtung und Rührer werden 0.02 mol vinyloges Thiocarbonsäureamide V, 0,012 mol 1,4-Bibrombutan-2,3-dion und 60ml Acetanhydrid suspendiert und eine Stunde am Rückfluß erhitzt. Danach wird abgekühlt, abgesaugt und getrocknet. Ausbeute beträgt mindestens 60%.

### Beispiel 11

Auf diese Art wird die Bisthienyl-1,2-dicarbonyl-verbindung VI/1 (R¹ = Phenyl, R² = Naphthyl, R³ = 4-O-Acetylphenyl M⁺ = 924 aus V/1 und 1,4-Dibromdiacetyl hergestellt.

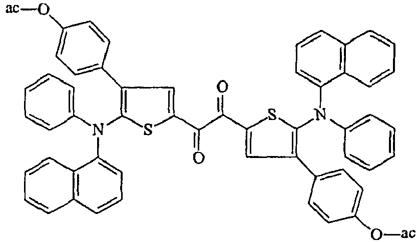

### Beispiel 12

Auf diese Art wird die Poly-bisthienyl-1,2-dicarbonylverbindung VI/2 (R¹ = 1,4-Phenylen, R² = Phenyl, R3 = 4-O-Acetylphenyl aus V/2 und 1,4-Dibromdiacetyl hergestellt.

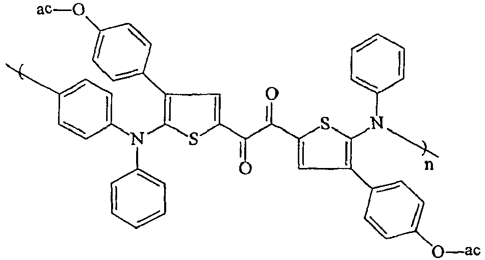

### h) Allgemeine Vorschrift zur Synthese der 5,6-annelierten 2,3-Bisthienyl-pyrazine VII

In einem Kolben mit Rückflusskühler, Heizeinrichtung und Rührer werden 0.02 mol Bisthienyl-1,2-dicarbonyl-verbindung VI, 0,012 mol 1,2-Phenylendiamin, 60ml Dimethylformamid und 1 ml konzentrierte Salzsäure suspendiert und eine Stunde am Rückfluß erhitzt. Danach wird abgekühlt, abgesaugt und getrocknet. Ausbeute beträgt mindestens 60%. Die Chromophore fallen nach der Reaktion bereits in ihrer ungeschützten Form an.

### Beispiel 13

Auf diese Art wird das 2,3-Bisthienyl-chinoxalin VII/1 (R¹ = Phenyl, R² = Naphthyl, R3 = 4-Hydroxyphenyl M⁺ = 912 λₘₐₓ = 410 nm, λ_{Φ} = 563 nm aus VI/1 und 1,2-Phenylendiamin hergestellt.

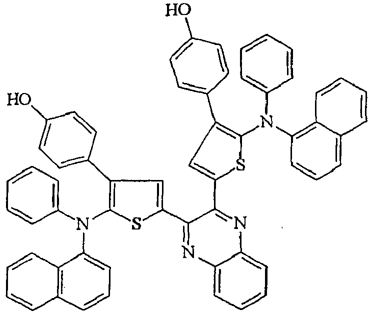

### Beispiel 14

Auf diese Art wird das Poly-2,3-bisthienyl-chinoxalin VII/2 (R¹ = 1,4-Phenylen, R² = Phenyl, R3 = 4-Hydroxyphenyl aus VI/2 und 1,2-Phenylendiamin hergestellt.

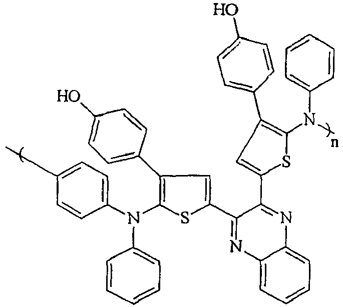

### i) Allgemeine Vorschrift zur Synthese der Arylketone VIII

0,1 mol des jeweiligen vinylogen Thiocarbonsäureamids V werden in einem Becherglas zusammen mit der äquivalenten Menge Dibromacetylbenzol in 150 ml Acetanhydrid 30 min unter Rühren auf 120°C erwärmt; dabei entsteht eine klare Lösung, aus der nach dem Erkalten das gebildete Arylketon VIII ausfällt. Die Reaktionsmischung wird abgesaugt, danach wird Produkt in THF gelöst und in Methanol gefällt. Die Ausbeute beträgt jeweils mindestens 80%.

### Beispiel 15

Auf diese Art wird das Aryklketon VIII/1 (R¹ = Phenyl, R² = Naphthyl, R3 = 4-O-Acetylphenyl M⁺ = 1000 aus V/1 und 1,4-Dibromacetylbenzol hergestellt.

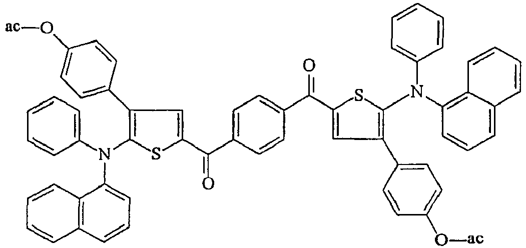

### Beispiel 16

Auf diese Art wird das Poly-arylketon VIII/2 (R¹ = 1,4-Phenylen, R² = Phenyl, R3 = 4-O-Acetylphenyl aus V/2 und 1,4-Dibromacetylbenzol hergestellt.

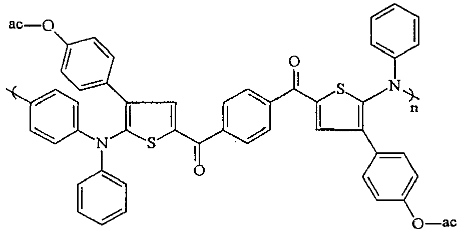

### j) Allgemeine Vorschrift zur Synthese der 1,4-Bisstyrylbenzenderivate IX

In einem Dreihalskolben, der mit Inertgasdurchfluss, Rückflusskühler und Feststoffdosiereinrichtung versehen ist, werden 0,01 mol des jeweiligen Aryklketon-Derivates VIII und die äquivalente Menge Phosphonsäureester in einem Gemisch aus 100 ml trockenem Toluol und 100 ml trockenem THF gelöst und bei 80°C gerührt. Danach wird zur Deprotonierung des Phosphonsäureesters jeweils die äquivalente Menge Kalium-tert.-butylat mittels der Feststoffdosiereinrichtung zugegeben. Gewöhnlich fällt dabei das Produkt bereits in der Wärme aus. Das Ende der Reaktion kann dünnschichtchromatographisch detektiert werden. Zur Aufarbeitung wird der Reaktionsmischung die 2fache Menge Methanol zugesetzt, und dann wird durch Zugabe von Eisessig neutralisiert. Anschließend wird das erhaltene Produkt abgesaugt, mit Methanol gewaschen und getrocknet. Durch Lösen in THF und wiederholtem Ausfällen mit Methanol wird das Produkt gereinigt. Die Rohausbeute des halbleitenden Materials ist jeweils nahezu quantitativ. Ein Teil des Produktes liegt bereits in der Hydroxyform vor.

### Beispiel 17

Auf diese Art wird das 1,4-Bisstyryl-benzenderivat IX/1 (R¹ = Phenyl, R² = Naphthyl, R3 = 4-O-Acetylphenyl M⁺ = 1148, λₘₐₓ = 395nm, λ_{Φ} = 479nm aus VIII/1 und Phenylmethanphosphonsäurediethylester hergestellt.

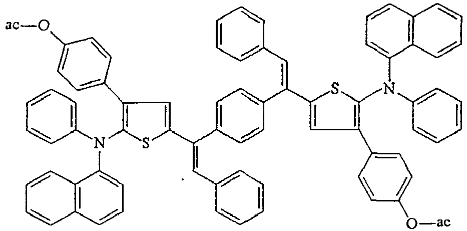

### Beispiel 18

Auf diese Art wird das Poly-(1,4-Bisstyryl-benzen)-derivat IX/2 (R¹ = 1,4-Phenylen, R² = Phenyl, R3 = 4-O-Acetylphenyl aus VIII/2 und Phenylmethanphosphonsäurediethylester hergestellt.

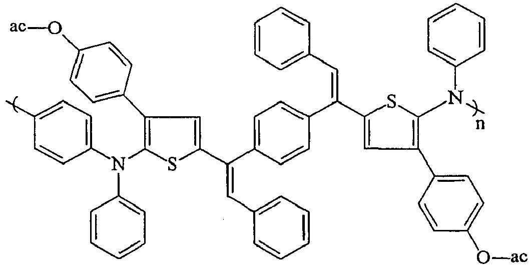

### k) Allgemeine Vorschrift zur Synthese der 1,1-Bisthienyl-2,2-bisakzeptorethylene X

In einem 100 ml Kolben mit Rührer, Heizeinrichtung und Rückflußkühler werden 0,005 mol 1,1-Di-(brommethyl)-2,2-dicyano-ethylen zusammen mit 0,01 mol des jeweiligen vinylogen Thiocarbonsäureamids V in 50 ml THF gelöst und für 10h am Rückfluß erhitzt. Nach beendeter Reaktion wird abgekühlt, vom entstandenen Nebenprodukt (Morpholiniumhydrobromid) abgesaugt und am Rotationsverdampfer eingeengt. Schließlich erfolgt säulenchromatographische Reinigung des Produktes über Kieselgel. Die Ausbeute beträgt mindestens 40%.

### Beispiel 19

Auf diese Art wird das 1,1-Bisthienyl-2,2-biscyanorethylene X/1 (R¹ = Phenyl, R² = Naphthyl, R3 = 4-O-Acetylphenyl M⁺ = 944, λₘₐₓ = 490nm, λ_{Φ} = 612 nm aus dem vinylogen Thiocarbonsäureamid V/1 und 1,1-Di-(brom-methyl)-2,2-dicyano-ethylen hergestellt.

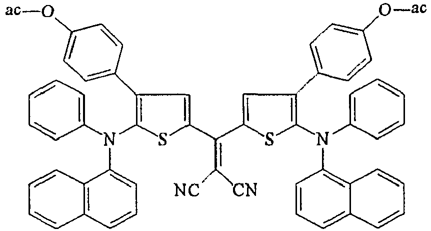

### 1) Allgemeine Vorschrift zur Synthese der 5-akzeptorsubstituierten Aminothiophene XI

In einem 100 ml Kolben mit Rührer, Heizeinrichtung und Rückflußkühler werden 0,012 mol 1-(Brommethyl)-1-methyl-2,2-dicyano-ethylen zusammen mit 0,01 mol des jeweiligen vinylogen Thiocarbonsäureamids V in 50 ml THF gelöst und für 10h am Rückfluß erhitzt. Nach beendeter Reaktion wird abgekühlt, vom entstandenen Nebenprodukt (Morpholiniumhydrobromid) abgesaugt und am Rotationsverdampfer eingeengt. Schließlich erfolgt säulenchromatographische Reinigung des Produktes über Kieselgel. Die Ausbeute beträgt mindestens 40%.

### Beispiel 20

Auf diese Art wird das 1-[2-(N-phenyl-N-1-naphthylamino-3-{4-O-acetyl}-phenyl)-thiophen-5-yl]-1-methyl-2,2-dicyano-ethylen XI/1 (R¹ = Phenyl, R² = Naphthyl, R3 = 4-O-Acetylphenyl M⁺ = 525, λₘₐₓ = 480nm, λ_{Φ} = 590 nm aus dem vinylogen Thiocarbonsäureamid V/1 und 1-(Brommethyl)-1-methyl-2,2-dicyano-ethylen hergestellt.

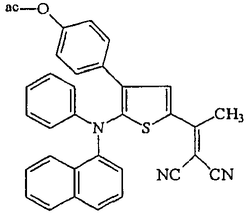

### m) Allgemeine Vorschrift zur Synthese der 4,6-Bisthienyl-1,3,2-Dioxaborine XII

Eine Mischung aus einem vinylogen Thiocarbonsäureamid V (0, 002 mol) und 1,5-Dichlor-pentan-2,4-dion (0,001 mol) in Acetonitril (50 mL) wird 30min auf dem Wasserbad erwärmt. Das beim Erkalten nach Zugabe von Wasser (10 ml) ausfallende Produkt wird abgesaugt und an der Luft getrocknet. Eine Mischung des isolierten 1,3-Diketoderivats (0,001 mol) in Acetanhydrid (100 mL) wird mit einem Überschuß eines Borsäure-Derivates versetzt und anschließend 30 min unter Rückfluß erhitzt. Nach dem Erkalten wird vom ausgefallenem Produkt abgesaugt und zur Reinigung aus Acetanhydrid umkristallisiert.

### Beispiel 21

Auf diese Art wird das 4,6-Bisthienyl-1,3,2-Dioxaborine XII (R¹ = Phenyl, R² = Naphthyl, R3 = 4-O-Acetylphenyl M⁺ = 986, λₘₐₓ = 570nm, λ_{Φ} = 605nm aus dem vinylogen Thiocarbonsäureamid V/1, 1,5-Dichlor-pentan-2,4-dion und Bortrifuorid-Etherat hergestellt.

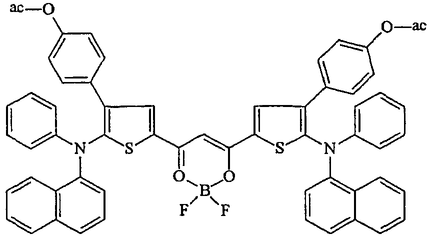

### n) Allgemeine Vorschrift zur Entfernung der Schutzgruppen an den Chromophoren II, IIa, IV, VII, IX, X, XI und XII

### Beispiel 22

In einem Becherglas werden 0,01 mol des jeweiligen Chromophors in möglichst wenig DMF bei 50°C gelöst und nach Zugabe von 5 ml conc. Ammoniaklösung bei gerührt. Der Fortschritt der Reaktion wird dünnschichtchromatographisch kontrolliert. Sollte sich nach 30 min keine wesentliche Umsetzung vollzogen haben wird die Reaktionstemperatur schrittweise erhöht.

Nach vollständigem Umsatz wird abgekühlt und in Methanol gefällt, mit Ammoniak neutralisiert, abgesaugt und getrocknet.

### o) Synthese kupplungsfähiger Oxetanderivate

### Beispiel 23

### 3-Ethyl-3-hydroxymethyloxetan:

In einem 11 Kolben werden 536g (4 mol) 1,1,1-Tris-(hydroxymethyl)-propan in 472g (4 mol) Diethylcarbonat durch Erwärmen gelöst. Anschließend werden 0,5g KOH gelöst in 20ml Ethanol zugegeben und die Reaktionsmischung 3h unter Rückfluß erhitzt. Durch Austausch des Kühlers durch einen Wasserabscheider wird danach der gebildete Alkohol vollständig abdestilliert. Abschließend destilliert man das Reaktionsprodukt im Vakuum.

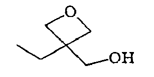

### Beispiel 24

### 3-Ethyl-3-chlormethyloxetan:

In einem Dreihalskolben mit Rückflußkühker und Inertgasdurchfluß werden 134g (1 mol) 1,1,1-Tris-(hydroxymethyl)-propan in 2 mol Pyridin gelöst. Unter Rühren und Kühlung werden langsam 2 mol Thionylchlorid zugetropft. Die Lösung wird danach für 12h am Rückfluß erhitzt. Nach dem Abkühlen fügt man der Lösung jeweils 400ml Wasser und Ether zu und trennt die Etherphase im Scheidetrichter ab. Dann wird die Etherphase mit Wasser gewaschen, über Magnesiumsulfat getrocknet, am Rotationsverdampfer eingeengt und das Produkt 1,1-Bis-(chlormethyl)-1-hydroxymethyl-propan schließlich am Vakuum destilliert.

0,5 mol dieser Verbindung werden in 250ml abs. Ethanol mit 28g KOH für 10h am Rückfluß gekocht. Nach dem Abkühlen filtriert man vom KCl ab, fügt dem Filtrat jeweils 100ml Wasser und Ether zu und isoliert die etherische Phase. Die Etherphase wird nun nochmals mit Wasser ausgeschüttelt, über Magnesiumsulfat getrocknet, der Ether abgezogen und abschließend der Rückstand im Vakuum destilliert.

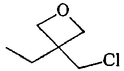

### Beispiel 25

### Allgemeine Vorschrift zur Veretherung des 3-Ethyl-3-chlormethyloxetans mit α,ω-Dihydroxyalkanen

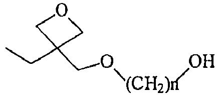

In einem Becherglas werden 0,5 mol eines α,ω-Alkandiols, 0,5 mol KOH, 0,05 mol KI und 0,5 mol 3-Ethyl-3-chlorethyl-oxetan gegeben und 6h auf 110°C erwärmt. Nach dem Abkühlen werden 350ml Ether zugegeben, vom Feststoff abfiltriert und der Filterrückstand mit 500ml Ether gewaschen. Nun wird der Ether am Rotationsverdampfer entfernt und das Produkt im Vakuum destilliert.
i) Auf diese Art wird beispielsweise aus 1,6-Hexandiol und 3-Ethyl-3-chlormethyloxetan die folgende verlängerte Oxetanverbindung hergestellt.

### p) Allgemeine Vorschrift zur Synthese vernetzend wirkender Lösungsmittel / Verdünner

### Beispiel 26

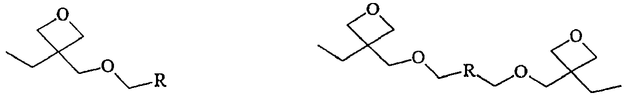

In einer Rückflußapparatur werden 120g 50%-ige NaOH, 120ml Hexan, 2g Tetrabutylammoniumbromid und 0,1 mol Chlormethyl- bzw. Hydroxymethyloxetan-derivat vorgelegt. Zu dieser Mischung wird jeweils die äquivalente Menge einer entsprechenden Hydoxyalkyl- bzw. Halogenalkylverbindung zugefügt. (Im Falle des Einsatzes von chlorsubstituierten Edukten müssen zusätzlich 10 mol% Kaliumjodid zugefügt werden.) Die Reaktionsmischung wird 5h am Rückfluß gekocht. Nach Abkühlung werden 0,51 Wasser hinzugefügt und dreimal mit 200ml Ether extrahiert. Die vereinigten Etherphasen werden über Magnesiumsulfat getrocknet, der Ether am Rotationsverdampfer abgezogen und der Rückstand im Vakuum destilliert.
i) Auf diese Art wird beispielsweise aus α,α-Dibrommethylbenzol-1,4 und 3-Ethyl-3-hydroxymethyloxetan die folgende Bis-oxetanverbindung hergestellt. 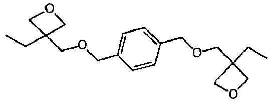
ii) Auf diese Art wird beispielsweise aus Benzylbromid und 3-Ethyl-3-hydroxymethyloxetan die folgende Mono-oxetanverbindung hergestellt. 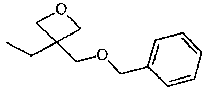
iii) Auf diese Art wird beispielsweise aus Pentaerytrit und 3-Ethyl-3-chlormethyloxetan die folgende Tetra-oxetanverbindung hergestellt, die nur im Vakuum bei Drücken unter 10⁻⁵ mb destillierbar ist.

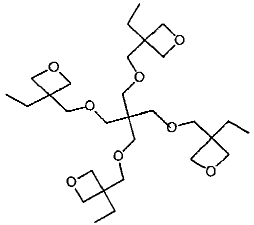

### q) Allgemeine Vorschrift zur Synthese der oxetan-funktionalisierten CTM

### Beispiel 27

30 mmol einer ungeschützten chromophoren Verbindung II, IIa, IV, VII, IX, X, XI oder XII, nach Beispiel 22, werden unter Rühren, Feuchtigkeitsausschluß und Inertgasspülung mit 30mmol kupplungsfähiger Oxetanverbindung, nach Beispiel 25 bzw. 23, und 60 mmol) Triphenylphosphin in 250ml trockenem THF (trockenem NMP (N-Methyl-pyrolidon) bei polymeren Chromophoren) gelöst. Danach werden 60 mmol Azodicarbonsäurediethylester, gelöst in 50ml THF, langsam bei Raumtemperatur zugetropft. Zur Vervollständigung des Umsatzes wird weitere 8h bei 30°C gerührt. Schließlich werden die oxetanfunktionalisierten CTM durch Eintropfen in rührendes Methanol gefällt (eventuell Zugabe von etwas Wasser), abgesaugt und getrocknet. Das dabei erhaltene polymere Rohprodukt wird durch Zusatz von THF vollständig gelöst und wiederholt zur Reinigung in Methanol umgefällt. Die Rohprodukte der nichtpolymeren oxetanfunktionalisierten CTM werden säulenchromatographisch auf Kieselgel gereinigt.

### r) Fertigung einer OLED durch vernetzte Schichten

### Beispiel 28

- Substratvorbereitung (Reinigung und ITO-Strukturierung [ITO = Indium-Zinn-Oxid])
- Herstellen eines Lackes aus einem vernetzbaren Lösungsmittel, nach Beispiel 26, einem oxetanfunktionalisierten Lochtransportmaterial mit Het = II oder IIa, nach Beispiel 27, und 1 Ma% Photoinitiator (bezogen auf die Masse oxetanfunktionalisierter Verbindungen) z.B. 4-(Thiophenoxyphenyl)-diphenylsulfoniumhexafluoroantimonat, (eventuell auch Zusätze anderer Lösungsmittel um Druck-, Rakel- bzw. Spincoating-parameter einzustellen)
- Aufbringen einer oxetanfunktionalisierten Lochtransportschicht auf das Substrat
- Entfernen des Lösungsmittels im Vakuum
- Auflegen einer Belichtungsmaske
- Belichten mit UV-licht 300 nm (Belichtungszeit optimieren, abhängig von Abstand und Leistung), bei der Beschichtung der Substrate muß die Schichtdicke so optimiert werden, dass sie nach der Vernetzung die gewünschte Höhe (zwischen 50nm und 100nm) hat.
- Mit einem geeigneten Lösungsmittel (Aceton, Chloroform, THF) die von der Maske abgedeckten Bereiche des Substrates auf einem Spincoater wegspülen.
- kurzes Trocknen im Vakuum
- herstellen eines Lackes aus einem vernetzbaren Lösungsmittel, nach Beispiel 26, einem oxetanfunktionalisierten E-lektronentransportmaterial welches gleichzeitig Emittereigenschaften besitzt mit Het = IV, VII, IX, X, XI oder XII, nach Beispiel 27, und 1 Ma% Photoinitiator (bezogen auf die Masse oxetanfunktionalisierter Verbindungen) z.B. 4-(Thiophenoxy-phenyl)-diphenylsulfoniumhexafluoroantimonat, (eventuell auch Zusätze anderer Lösungsmittel um Druck-, Rakel- bzw. Spincoating-parameter einzustellen), zusätzlich können dem Lack auch andere nicht vernetzbare und /oder vernetzbare Emittermaterialien ebenso wie andere nicht vernetzbare und/oder vernetzbare Elektronentransportmaterialien beigemischt werden, um gewünschte Emissionseigenschaften und Elektronentransportegenschaften zu erzeugen.
- Aufbringen einer oxetanfunktionalisierten Elektronentransportschicht auf das Substrat, welche gleichzeitig Emittereigenschaften besitzt.
- Entfernen des Lösungsmittels im Vakuum
- Auflegen einer Belichtungsmaske
- Belichten mit UV-licht 300 nm (Belichtungszeit optimieren, abhängig von Abstand und Leistung), bei der Mehrfachbeschichtung der Substrate muß die Schichtdicke so optimiert werden, dass sie nach der Vernetzung die gewünschte Gesamthöhe (bestehende Schichtdicke + 50nm bis 100nm) hat.
- Mit einem geeigneten Lösungsmittel (Aceton, Chloroform, THF) die von der Maske abgedeckten Bereiche des Substrates auf einem Spincoater wegspülen.
- Der Beschichtungsvorgang wird nun sooft wiederholt, bis die gesamte OLED-Fläche mit Elektronentransport- und/oder Emittermaterialien abgedeckt ist.
- Aufdampfen einer 40 nm dicken Ca-Kathode im Vakuum
- Hermetische Verkapselung der elektrooptischen Anordnung mit herausgeführten Elektrodenanschlüssen.
- Spannung anlegen - Lumineszenz

Stromdichte und Leuchtdichte für eine OLED mit folgender Schichtanordnung:
- ITO-Glassubstrat
- 60 nm Lochtransportschicht II nach Beispiel 4
- 60nm Chromophorschicht IX nach Beispiel 17
- 40nm Ca

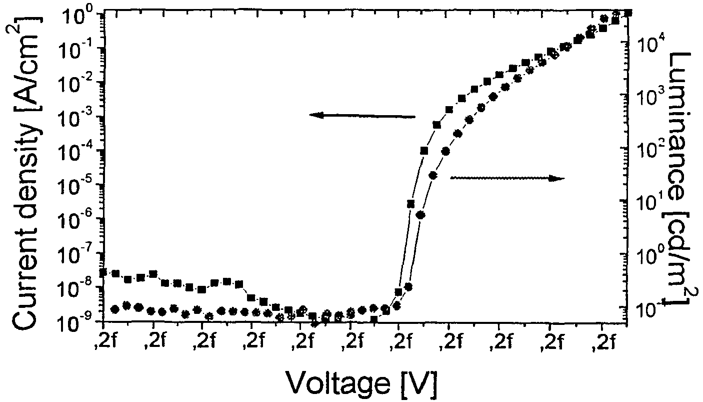

## Patentansprüche

1. Niedermolekularer, oligomerer oder (pre)polymerer Halbleiter mit Oxetan-funktionalisiertem Substituenten, wobei der Halbleiter ladungstransportierende und/oder lumineszierende Eigenschaften hat und durch thermische Behandlung und/oder durch Bestrahlung vernetzbar ist.

2. Halbleiter nach Anspruch 1 der folgenden allgemeinen Struktur: 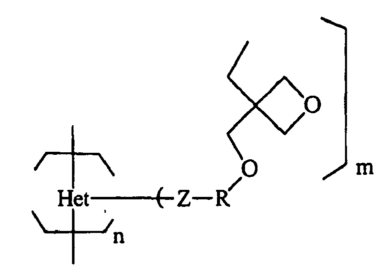 für das folgendes gilt:
**Het** sind die heterocyclischen Grundkörper der ladungstransportierenden Verbindungen,
**Z** sind die Substituenten **R³** und/oder **R⁴** der heterocyclischen Grundkörper, die unabhängig voneinander oder beide eine Funktion tragen, die zur Anknüpfung an den Spacer **R** geeignet ist,
die Gruppe der einsetzbaren Spacer **R** umfasst verzweigte oder unverzweigte, unsubstituierte Alkylenketten, vorzugsweise C₁ bis C₈ -Ketten, und/oder etherhaltige Alkylenketten und/oder aromatenhaltige Alkylenketten,
die Zahl der Repititionseinheiten **n** umfaßt die Werte 1 bis 100, und
die Anzahl der vernetzbaren Gruppierungen **m** pro Repititionseinheit umfaßt die Werte 1 und 2.

3. Halbleiter nach Anspruch 2, wobei die Gruppe **Z-Het** ausgewählt ist aus der Gruppe, die folgende Verbindungen umfasst:
2-Aminothiophenderivate **II** und **IIa**
5-akzeptor-funktionalisierte 2-N,N-Diarylamino-thiophene **IV** 2,3-Bishtienylchinoxaline **VII**
1,4-Bis-1,1'-dithienyl-2,2'-diaryl-bzw.-2,2,2',2'-tetraarylstyrylbenzene **IX**
1,1-Dithienyl-2,2-diakzeptor-funktionalisierte Ethylene **X**
1-Thienyl-1-methyl-2,2-diakzeptor-funktionalisierte Ethylene **XX**
4,6-bisthienyl-funktionalisierte 1,3,2-Dioxaborine **XII.**

4. Halbleiter nach einem der vorstehenden Ansprüche, wobei der Spacer **R,** über den die Oxetan-Funktion durch eine Ethergruppierung kovalent gebunden ist, mit dem Oxetan-Ring vorzugsweise über eine Ether- oder Amingruppierung mit einem Arylrest R3 und/oder R4 der ladungstransportierenden Verbindung verknüpft ist, wobei sich folgende Möglichkeiten der Anbindung bieten: Phenolether, Benzylether, Phenolthioether, und/oder tertiäres Amin.

5. Verfahren zur Herstellung eines niedermolekularen, oligomeren oder (pre)polymeren Halbleiters mit Oxetan-funktionalisiertem Substituenten, durch Kondensation und/oder HX-Eliminierung nach folgendem allgemeinen Reaktionsschema: 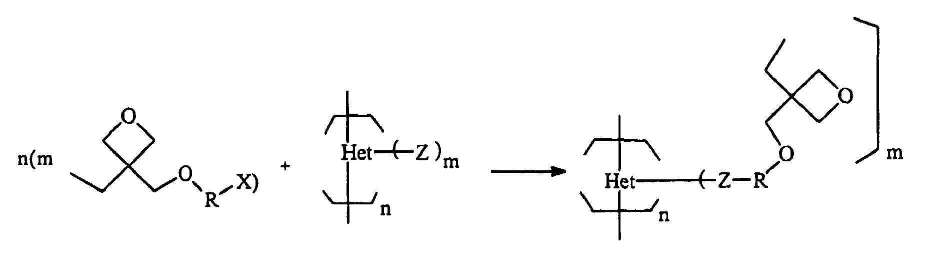 wobei **R, Z, Het n** und **m** die in Anspruch 2 angegebenen Bedeutungen haben und
**X** ein Halogen oder OH, je nach dem angewandten Reaktionstyp zur Verknüpfung von **Z** mit **R,** ist.

6. Verfahren zur Herstellung zumindest einer halbleitenden und/oder lumineszierenden Schicht durch Vernetzung eines niedermolekularen, oligomeren oder (pre)polymeren Halbleiters mit Oxetan-funktionalisiertem Substituenten.

7. Verfahren nach Anspruch 6, folgende Verfahrensschritte umfassend:
- Beschichtung eines Substrates durch ein Verfahren aus Lösung und/oder aus Substanz mit dem Halbleiter, der in Lösung oder in Substanz vorliegen kann,
- Vernetzung des entstandenen Films.

8. Verfahren nach einem der Ansprüche 6 oder 7, bei dem der Film vor der Vernetzung noch getrocknet wird.

9. Verfahren nach einem der Ansprüche 6 bis 8, bei dem der Halbleiter in Lösung aufgebracht wird und die Lösung weitere Komponenten wie Bindemittel, Reaktivverdünner, Vernetzer, thermischen Initiator und/oder Photoinitiator enthält.

10. Verfahren nach einem der Ansprüche 6 bis 9, bei dem die Vernetzung durch thermisch latente Lewis-Säuren, durch Photosäuren, Photoinitiatoren und/oder durch Bestrahlung mit elektromagnetischer und/oder aktinischer Strahlung initiiert wird.

11. Verfahren nach einem der Ansprüche 6 bis 10, bei dem eine Strahlung der Wellenlänge zwischen 200 nm und 600 nm eingesetzt wird.

12. Verfahren nach einem der Ansprüche 6 bis 11, durch das eine halbleitende Schicht mit einer Schichtdicke im Bereich von einigen Nanometern bis hin zu Millimetern hergestellt wird.

13. Verfahren nach einem der Ansprüche 6 bis 12, durch das eine Schicht im Bereich von 25 bis 250 nm hergestellt wird.

14. Verfahren nach einem der Ansprüche 6 bis 13, bei dem die halbleitende Schicht strukturiert wird.

15. Verfahren nach einem der Ansprüche 6 bis 14, bei dem die halbleitende und/oder lumineszierende Schicht durch bildgemäßes Belichten strukturiert wird.

16. Organische Licht emittierende Diode (OLED), umfassend zumindest ein Substrat, eine Anodenschicht, eine Lochtransportschicht, eine Emitterschicht, die gleichzeitig auch eine Elektronentransportschicht sein kann, eine Kathode und eine Verkapselung, wobei die Lochtransportschicht und/oder die Elektronentransportschicht/Emitterschicht durch Vernetzung eines niedermolekularen, oligomeren oder (pre)polymeren Halbleiters mit Oxetan-funktionalisiertem Substituenten erhältlich ist.

## Claims

1. Low molecular weight, oligomeric or (pre)polymeric semiconductor having oxetane-functionalized substituents, wherein the semiconductor has charge-transporting and/or luminescent properties and is crosslinkable through thermal treatment and/or through irradiation.

2. Semiconductor according to Claim 1 of the following general structure: 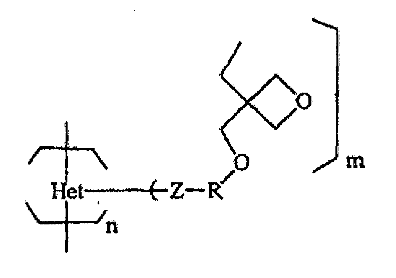 where:
**Het** are the heterocyclic base structures of the charge-transporting compounds,
**Z** are the substituents **R³** and/or **R⁴** of the heterocyclic base structures, which independently or both bear a function suitable for attachment to the spacer **R,**
the group of usable spacers **R** comprises branched or unbranched, unsubstituted alkylene chains, preferably C₁ to C₈ chains, and/or ether-containing alkylene chains and/or aromatic-containing alkylene chains,
the number of repeat units **n** encompasses the values of 1 to 100, and
the number of crosslinkable moieties **m** per repeat unit comprises the values of 1 and 2.

3. Semiconductor according to Claim 2, wherein the **Z-Het** group is selected from the group comprising the following compounds:
2-aminothiophene derivatives **II** and **IIa**
5-acceptor-functionalized 2-N,N-diarylaminothiophenes **IV** 2,3-bisthienylquinoxalines **VII**
1,4-bis-1,1,-dithienyl-2,2'-diaryl- or -2,2,2',2'-tetraarylstyrylbenzenes **IX**
1,1-dithienyl-2,2-diacceptor-functionalized ethylenes **X**
1-thienyl-1-methyl-2,2-diacceptor-functionalized ethylenes **XI**
4,6-bisthienyl-functionalized 1,3,2-dioxaborins **XII.**

4. Semiconductor according to any of the preceding claims, wherein the spacer **R** through which the oxetane function is covalently bonded by an ether moiety is joined to the oxetane ring preferably via an ether or amine moiety having an aryl radical R3 and/or R4 of the charge-transporting compound, with the following options for the attachment: phenol ether, benzyl ether, phenol thioether and/or tertiary amine.

5. Process for preparing a low molecular weight, oligomeric or (pre)polymeric semiconductor having oxetane-functionalized substituents, by condensation and/or HX elimination according to the following general reaction scheme: 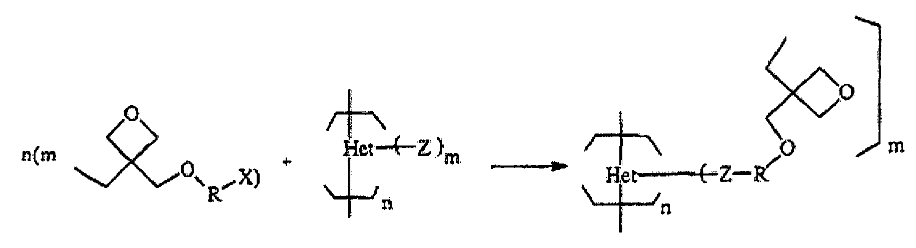 where **R, Z, Het, n** and **m** are each as defined in Claim 2 and
**X** is a halogen or OH, according to the type of reaction employed for joining **Z** to **R.**

6. Process for producing at least one semiconductive and/or luminescent layer by crosslinking a low molecular weight, oligomeric or (pre)polymeric semiconductor having oxetane-functionalized substituents.

7. Process according to Claim 6, comprising the following process steps:
- coating a substrate by a process from solution and/or from substance with the semiconductor which may be in solution or in substance,
- crosslinking the resultant film.

8. Process according to either of Claims 6 and 7, in which the film is dried prior to the crosslinking.

9. Process according to any of Claims 6 to 8, in which the semiconductor is applied in solution and the solution comprises further components such as binder, reactive diluent, crosslinker, thermal initiator and/or photoinitiator.

10. Process according to any of Claims 6 to 9, in which the crosslinking is initiated by thermally latent Lewis acids, by photoacids, by photoinitiators and/or by irradiation with electromagnetic and/or actinic radiation.

11. Process according to any of Claims 6 to 10, in which radiation of wavelength between 200 nm and 600 nm is used.

12. Process according to any of Claims 6 to 11, by which a semiconductive layer having a layer thickness in the range from a few nanometres up to millimetres is produced.

13. Process according to any of Claims 6 to 12, by which a layer in the range from 25 to 250 nm is produced.

14. Process according to any of Claims 6 to 13, in which the semiconductive layer is structured.

15. Process according to any of Claims 6 to 14, in which the semiconductive and/or luminescent layer is structured by imagewise exposure.

16. Organic light-emitting diode (OLED) comprising at least a substrate, an anode layer, a hole transport layer, an emitter layer which may simultaneously also be an electron transport layer, a cathode and an encapsulation, wherein the hole transport layer and/or the electron transport layer/emitter layer is obtainable by crosslinking a low molecular weight, oligomeric or (pre)polymeric semiconductor having oxetane-functionalized substituents.

## Revendications

1. Semi-conducteur de bas poids moléculaire, oligomère ou (pré)polymère présentant des substituants fonctionnalisés par oxétane, le semi-conducteur présentant des propriétés de transport de charges et/ou luminescentes et étant réticulable par traitement thermique et/ou par irradiation.

2. Semi-conducteur selon la revendication 1, présentant la formule générale suivante : 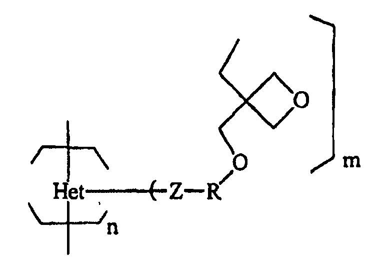 dans laquelle :
Het représente les corps de base hétérocycliques des composés de transport de charges,
Z représente les substituants R³ et/ou R⁴ des corps de base hétérocycliques, qui portent, indépendamment l'un de l'autre ou tous les deux, une fonction appropriée pour la liaison à l'espaceur R,
le groupe des espaceurs R utilisables comprend des chaînes alkylène ramifiées ou non ramifiées, non substituées, de préférence des chaînes en C₁ à C₈ et/ou des chaînes alkylène contenant éther et/ou des chaînes alkylène contenant des aromatiques, le nombre d'unités récurrentes n comprend les valeurs 1 à 100 et le nombre de groupements réticulables m par unité récurrente comprend les valeurs 1 et 2.

3. Semi-conducteur selon la revendication 2, le groupe Z-Het étant choisi dans le groupe comprenant les composés suivants :
les dérivés de 2-aminothiophène II et IIa
les 2-N,N-diarylaminothiophènes IV fonctionnalisés par un accepteur en position 5
les 2,3-bisthiénylquinoxalines VII
les 1,4-bis-1,1'-dithiényl-2,2'-diarylstyrylbenzènes ou les 1,4-bis-1,1'-dithiényl-2,2,2',2'-tétraarylstyrylbenzènes IX
les éthylènes X fonctionnalisés par 1,1-dithiényl-2,2-diaccepteur
les éthylènes XI fonctionnalisés par 1-thiényl-1-méthyl-2,2-diaccepteur
les 1,3,2-dioxaborines XII fonctionnalisées par 4,6-bisthiényle.

4. Semi-conducteur selon l'une quelconque des revendications précédentes, l'espaceur R, via lequel la fonction oxétane est liée par covalence par un groupement éther, étant lié au cycle oxétane de préférence via un groupement éther ou amine à un radical aryle R³ et/ou R⁴ du composé de transport de charges, les possibilités de liaison suivantes des liaisons existant : phénoléther, benzyléther, phénolthioéther et/ou amine tertiaire.

5. Procédé pour la préparation d'un semi-conducteur de bas poids moléculaire, oligomère ou (pré)polymère présentant des substituants fonctionnalisés par oxétane, par condensation et/ou élimination de HX selon le schéma de réaction général suivant : 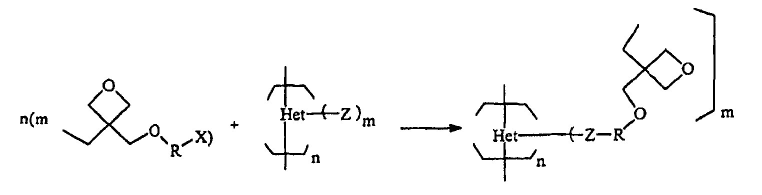 dans lequel R, Z, Het, n et m ont les significations indiquées dans la revendication 2 et
X représente un halogène ou OH, en fonction du type de réaction utilisé pour la liaison de Z avec R.

6. Procédé pour la préparation d'au moins une couche semi-conductrice et/ou luminescente par réticulation d'un semi-conducteur de bas poids moléculaire, oligomère ou (pré)polymère présentant des substituants fonctionnalisés par oxétane.

7. Procédé selon la revendication 6, comprenant les étapes de procédé suivantes :
- revêtement d'un substrat par un procédé à partir d'une solution et/ou de la masse par le semi-conducteur, qui peut se trouver en solution ou en masse,
- réticulation du film formé.

8. Procédé selon l'une quelconque des revendications 6 ou 7, dans lequel le film est encore séché avant la réticulation.

9. Procédé selon l'une quelconque des revendications 6 à 8, dans lequel le semi-conducteur est appliqué en solution et la solution contient d'autres composants tels que des liants, des diluants réactifs, des réticulants, des initiateurs thermiques et/ou des photo-initiateurs.

10. Procédé selon l'une quelconque des revendications 6 à 9, dans lequel la réticulation est initiée par des acides de Lewis thermiquement latents, par des photoacides, des photo-initiateurs et/ou par irradiation par un rayonnement électromagnétique et/ou actinique.

11. Procédé selon l'une quelconque des revendications 6 à 10, dans lequel on utilise un rayonnement de longueur d'onde entre 200 nm et 600 nm.

12. Procédé selon l'une quelconque des revendications 6 à 11, qui permet de préparer une couche semi-conductrice présentant une épaisseur de couche dans la plage de quelques nanomètres jusqu'à quelques millimètres.

13. Procédé selon l'une quelconque des revendications 6 à 12, qui permet de préparer une couche dans la plage de 25 à 250 nm.

14. Procédé selon l'une quelconque des revendications 6 à 13, dans lequel la couche semi-conductrice est structurée.

15. Procédé selon l'une quelconque des revendications 6 à 14, dans lequel la couche semi-conductrice et/ou luminescente est structurée par éclairage selon une image.

16. Diode électroluminescente organique (DELO), comprenant au moins un substrat, une couche d'anode, une couche de transport de trous, une couche émettrice qui peut simultanément aussi être une couche de transport d'électrons, une cathode et une encapsulation, la couche de transport de trous et/ou la couche de transport d'électrons/couche émettrice pouvant être obtenue(s) par réticulation d'un semi-conducteur de bas poids moléculaire, oligomère ou (pré)polymère présentant des substituants fonctionnalisés par oxétane.
